# EUROPEAN PATENT APPLICATION

(11) **EP 2 722 049 A1**
(43) Date of publication of application: **23.04.2014**
(21) Application number: 11867128.8
(22) Date of filing: 09.06.2011
(51) Int. Cl.: A61K 31/7024, A61P 35/04

(54) **A PHARMACEUTICAL COMPOSITION FOR INHIBITING RECURRENCE, AGGRAVATION AND METASTASIS OF HEPATOCARCINOMA**

(71) Applicant: Medigen Biotechnology Corp., Taipei City (TW)
(72) Inventor: CHANG, Stanley, Taipei City Taiwan (TW); LAI, Kuan-Lang, Taipei City Taiwan (TW)
(74) Representative: Goddar, Heinz J.
(86) International application number: PCT/CN2011/075512
(87) International publication number: WO 2012/167434

(57) **Abstract**

The present application provides a pharmaceutical composition for use in inhibiting recurrence, aggravation or metastasis of liver cancer, in which the pharmaceutical composition comprises at least one compound having a structure of formula (I) and a pharmaceutically acceptable vehicle.

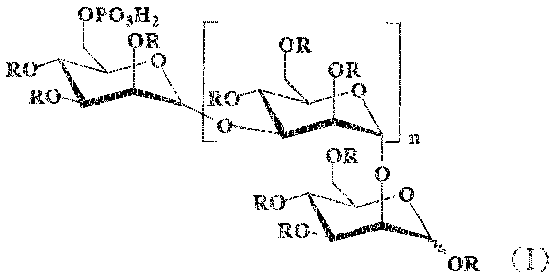

## Description

### FIELD OF THE INVENTION

The present invention relates to a pharmaceutical composition, specifically a pharmaceutical composition for use in inhibiting liver cancer recurrence, aggravation or metastasis. The present invention also relates to a method of preparing a medicament, especially for a method of preparing a medicament for use in inhibiting liver cancer recurrence, aggravation or metastasis.

### BACKGROUND OF THE INVENTION

According to the International Journal of Cancer, liver cancer is the fifth most common malignant tumor. The most common form of liver cancer is hepatocellular carcinoma (HCC), which accounts for approximately 70 ∼ 85 percent of all liver cancer patients. Currently, treatments including surgical resection, local ablation and liver transplant surgery are effective for only 15% to 20% of the patients. Although early diagnosis and treatment could increase survival rates, patients receiving surgical resection usually experienced recurrence of liver cancer. The data in Annals of Surgery indicate that 60% of the liver cancer patients developed cancer recurrence within 12 months after surgical resection. Liver cancer micro-metastasis can be detected at the time of surgery with sensitive detection technology; however, micro-metastasis detected in the patient still had 30% to 40% of liver cancer recurrence rate within a year after the surgery. In addition, a residual pathological liver tissue left behind the surgery is also likely to re-form liver tumors. Recurrence of liver cancer substantially shortens the overall survival time of the patients. Therefore, how to prevent or delay liver cancer recurrence after surgical resection is a major problem that remains to be solved.

In the past, many tests were conducted to find effective methods for decreasing the recurrence rate of liver cancer, for example, trans-arterial chemotherapy, retinoids, adjuvant interferon, adoptive immunotherapy, and intra-arterial radioactive lipiodol, etc. Although the above methods have potential to decrease the risk of liver cancer recurrence or increase the survival rate of the patients, they did not pass clinical trials and have not become a standard adjunctive therapy after liver resection. Therefore, there is still an urgent need for new drugs or new therapies.

Analyzing from the point of cancer formation, liver cancer is characterized by a high degree of blood vessel formation, and its development is greatly related to angiogenic factors. Researchers including Judah Folkman, an authority in the field of angiogenesis, have pointed out from the theory and experiments that a combination of anti-angiogenic drugs with other therapies can achieve a better therapeutic effect in treating cancer (Christopher Rice, L Eric Huang. From antiangiogenesis to hypoxia: current research and future directions. Cancer Management and Research. 2011:3 9-16). In addition, degradation of heparan sulfate (HS), which is a component of the extracellular matrix (ECM), has been proved to be a key factor related to tumor invasion and metastasis. One of the major enzymes participating in the degradation is heparanase.

Heparanase is an endogenous glucuronidase, which degrades the side chain of heparan sulfate in the extracellular matrix, and is the only endogenous glucuronidase that can degrade heparan sulfate proteoglycan (HSPG). It degrades heparan sulfate at a specific site and thereby promotes tumor invasion and metastasis. Heparanase makes a key impact in tumor metastasis and angiogenesis through degradation of extracellular matrix, release of angiogenic factors, and vascular remodeling process. In addition, heparan sulfate after degradation releases bioactive angiogenic factors into extracellular matrix, which can promote growth of blood vessels and thereby further cancer cell proliferation, metastasis, and aggravation. Therefore, inhibition of heparanase may be effective in suppressing tumor growth and metastasis. Whether heparanase is overexpressed or not becomes an important aim of prevention or treatment of malignant tumors.

Previous research found that heparanase in many types of tumors exhibited a higher level of expression, and is also related to the development of pathogenesis, leading to many research reports focusing on inhibition of this enzyme as a cancer therapeutic strategy, which includes development of small molecule drugs, chemically modified natural compounds, and neutralization antibodies, etc.

US patent No. 6,143,730 discloses preparation and use of a sulfated oligosaccharide. The oligosaccharide has a structure of formula I: R1-(Rx)n-R2, in which R1 and R2 and each Rx are a monosaccharide unit, all of which may be the same or different, the adjacent monosaccharide units being linked via 1→2, 1→3, 1→4 and/or 1→6 glycosidic bonds, and n is an integer of from 1 to 6, and the use thereof as anti-angiogenesis, anti-metastasis and/or anti-inflammatory agents. The aforementioned anti-angiogenesis effect had been demonstrated by *in vitro* and clinical trial data. The anti-metastasis effect has only animal data but not human clinical trial data.

In summary, although methods for decreasing risk of liver cancer recurrence or increasing the survival rate of patients are currently available, they have not been validated by data from human clinical trials with a sufficient patient number. Therefore, safe and effective new pharmaceutical compositions and therapeutic methods are in need.

### SUMMARY OF THE INVENTION

In one aspect, the invention provides a pharmaceutical composition for use in inhibiting recurrence, aggravation and/or metastasis of liver cancer, which comprises a therapeutically effective compound of formula (I) and a pharmaceutically acceptable diluent, excipient or 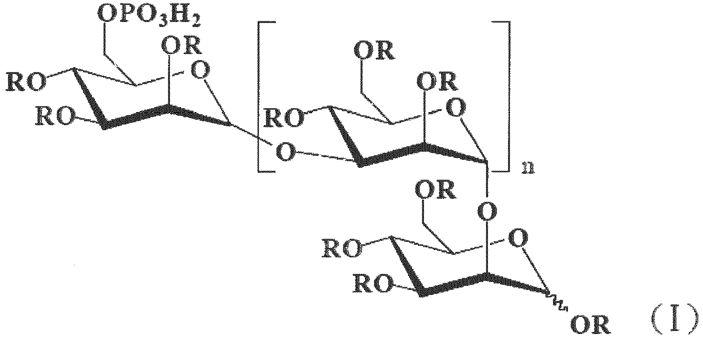 vehicle:
wherein in the compound of formula (I) n is an integer from 0 to 3, and 3n+6 or 3n+7 of R groups represent SO₃H, and the remaining R groups represents H.

According to the above, the compound of formula (I) having 3n+7 of R groups representing SO₃H is the major constituent (or component) of the aforementioned composition.

According to the above, the compound of formula (I) that has n equal to 2 or 3 and 3n+7 of R groups representing SO₃H is the major constituent (or component) of the aforementioned composition.

According to the above, the compound of formula (I) that has n equal to 3 and 3n+7 of R groups represent SO₃H has the highest relative content in the aforementioned composition.

According to the above, the effective therapeutic amount is between 80 mg/day to 315 mg/day. A preferred effective therapeutic amount is 160 mg/day.

As an active constituent of the pharmaceutical composition of the invention, the compound of formula (I) is present and used in a sodium salt form. It may be present and used in other pharmaceutically acceptable salt forms, e.g., in a calcium salt or ammonium salt form. Therefore, the active constituent of the pharmaceutical composition of the invention comprises a sodium salt or any other pharmaceutically acceptable salt of the compound of formula (I).

The pharmaceutically acceptable diluent, excipient or vehicle includes solvents, dispersing agents, filling agents (or bulking agent), solid vehicles, aqueous solutions, antibacterial agents, antifungal agents, and absorption-delaying agents, or analogous of the aforementioned agents. The composition of the invention may use compatible agents or test reagents, except those that are incompatible with the active constituents.

The aforementioned pharmaceutical composition may combine with at least one other therapy for use in inhibiting recurrence, aggravation or metastasis of liver cancer. The one other therapy comprises embolization therapy, target drug therapy, chemotherapy, radiation therapy and liver surgery resection.

According to the above, the pharmaceutical composition of the invention is for use in decreasing recurrence rate after the liver surgical resection or prolonging time to recurrence after the liver surgical resection in a liver cancer patient.

In another aspect, the invention provides a method of using a compound of formula (I) for preparing a medicament for use in inhibiting recurrence, aggravation or metastasis of liver cancer, comprising combining a therapeutically effective amount of the compound of formula (I) and a pharmaceutically acceptable diluent, excipient or vehicle.

According to the above, n is an integer of from 0 to 3, and 3n+6 or 3n+7 of the R groups are SO₃H, and the rest of the R groups are H.

According to the above, the compound having 3n+7 of the R groups as SO₃H is the primary constituent.

According to the above, the compound having n equal to 2 or 3 and 3n+7 of the R groups as SO₃H is the primary constituent.

According to the above, the compound having n equal to 3 and 3n+7 of the R groups as SO₃H has the highest relative content.

According to the above, the pharmaceutically acceptable diluent, excipient or vehicle is water or a water-solvent-based solution.

According to the above, the pharmaceutically acceptable diluent, excipient or vehicle is a physiological saline solution or a glucose solution.

The spirit of the invention will be more readily appreciated by one of ordinary skilled in the art from the following drawings, working examples and descriptions.

### BRIEF DESCRIPTION OF THE DRAWINGS

FIG. 1 is a LC/ESI-FTMS total ion chromatogram (TIC) of the active constituents of the pharmaceutical compositions according to the invention.
FIG. 2 shows the non-recurrence rate and dropout rate of the liver cancer patients tested in the control group, the experimental group receiving a daily dose of 160mg, and the experimental group receiving a daily dose of 250mg of the active constituent of the pharmaceutical composition of the invention.
FIG. 3 shows comparisons of the changes in the non-recurrence rates in liver cancer patients tested during a test period (0∼ 48 weeks) between the control group and the experimental group receiving a daily dose of 160mg of the active constituent of the pharmaceutical composition of the invention.

### DETAILED DESCRIPTION OF THE INVENTION

The invention "a pharmaceutical composition for use in inhibiting recurrence, aggravation and/or metastasis of liver cancer", and the spirit of its originality will be appreciated by ordinary skilled in the art and invention will be able to be practiced according to the following working examples and descriptions. The embodiments of the invention are not limited by the following working examples. Practice of the methods for preparation, validation and the use of the pharmaceutical composition of the invention are described as follows.

### Preparation of the Pharmaceutical Composition of the Invention

The active constituents of the pharmaceutical composition of the invention came from *Pichia holstii.* Its preparation requires steps of growing and fermentation of the yeast, hydrolysis of the product, purification and sulfonation as described below.

*Pichia holstii* NRRL Y-2448 were grown aerobically and nitrogen-limited environment in a culture medium that used D-glucose as the carbon source and contained an excess of orthophosphate. The phosphomannan (PS) secreted to extracellular medium contained a variety of polysaccharide structures, and was used as a basic starting material for semi-synthesis of the pharmaceutical composition of the invention. The phosphomannan (PS) is composed of a highly branched, high molecular weight (5 × 10⁶ ∼ 39 × 10⁶ Dalton) phosphomannan core (PC). The growing of P. *holstii* culture, fermentation and phosphomannan isolation was according to Jeanes, A.; Pittsley, J. E.; Watson, P. R.; Dimler, R. J. Arch. Biochem. Biophys. 1961, 92, 343-350, and Bretthauer, R. K.; Kaczorowski, G. J.; Weise, M. J. Biochemistry 1973, 12, 1251-1256. Generally, the yield was 20 kg of phosphomannan crude product per 400-L-scale fermentation.

In a preferred working example, the hydrolysis of phosphomannan was carried out at 100°C for 6-10 h at a concentration of about 40-50 g/L, pH 2.2-2.5 using 1 M HCl as a catalyst and in the presence of KCl. During the first hour of the reaction when the phosphomannan was mixed with the solution, the pH slightly rose to about pH 3, and thus was readjusted back to pH 2.2-2.5 by further additions of 1 M HCl. A single hydrolysis reaction processed about 2.5 Kg (wet weight) of phosphomannan. The hydrolysate, which contained relatively low molecular weight oligosaccharide phosphate fraction (OPF), was adjusted to pH 9∼9.5 by 1M NaOH.

In a more preferred working example, the solution for use in the hydrolysis reaction was prepared by dissolving KCl (600 g) in water (60 L) and acidified to pH 2.4 with 1 M HCl, and the resulting solution was placed in a stainless steel reaction vessel (75 L). Phosphomannan crude product (wet weight 2.49 kg) was added in portions to the solution via the addition portal, and the mixture was heated to 100 °C with vigorous stirring for 7 h. The pH was monitored hourly. One hour after the reaction had started, pH was readjusted with 1 M HCl to pH 2.3. After the hydrolysis was completed, the hydrolysate was cooled to the room temperature and the pH adjusted to pH 9.5 with 1 M NaOH.

After hydrolysis, purification was conducted by ultrafiltration using a membrane with a pore size of 10,000 Dalton (nominal molecular weight cut off membrane, NMWCO membrane). The oligosaccharide phosphate fraction, unphosphorylated oligosaccharides and salts in the hydrolysate permeated through the membrane whilst the phosphomannan core, which had a relatively high molecular weight, was retained on the membrane. Thus, the oligosaccharide phosphate fraction and the unphosphorylated oligosaccharides were separated from the phosphomannan core. The separation process was completed in diafiltration mode with a tangential or cross flow system. Such a system could scale up by increasing the available membrane area and the flow rate. A better membrane pore size ranges from 3,000 to 100,000 Dalton, and the best 10,000 Dalton.

In a preferred working example, the hydrolysate was diluted to 70 L and placed in a 150 L of a stainless steel tank. It was then diafiltered against eight diavolumes of purified water using an ultrafiltration system composed of two sets of Sartorius Hydrosart 10K (NMWCO 10,000) filter cassettes (each filter cassette's membrane area is 0.6 m²) connected in series. The set diafiltration parameters: inlet pressure 200 kPa, outlet pressure 150 kPa, retentate cross flow rate 15.6 L/min, permeate flux rate 66-87 L/h/m² (16-22 °C). The permeate (630 L, conductivity=1.1 mS/cm) was divided into six batches for processing by ion-exchange chromatography.

The above permeate was further purified with ion-exchange chromatography. In a better working example, the ion-exchange chromatography employed a column of 30 L DEAE-Spherilose equilibrated with 0.01 M NH₄HCO₃ at a flow rate of 1.5 L/min. The permeate (about 100 L per run, six runs) was loaded onto the column, and a neutral fraction was washed from the column with 0.01 M NH₄HCO₃ until the conductivity of the effluent was within 0.2 mS/cm of the baseline. Afterwards, the oligosaccharide phosphate fraction was eluted with 0.25 M NH₄HCO₃. One-liter fractions were collected and analyzed by HPLC. The appropriate fractions from six runs of chromatography were pooled together and concentrated to 20 L and the salts removed simultaneously by reverse osmosis. Reverse osmosis was continued in diafiltration mode until the conductivity of the permeate was ≤ 0.2 mS/cm, and the solution was further concentrated to a final volume of 6 L. After lyophilisation it was afforded an oligosaccharide phosphate fraction as a white, hygroscopic powder, about 566 g. The purity was about 93% as detected by HPLC, which was sufficiently pure for use in preparation of pharmaceutical composition of the invention without the need for further purification.

The aforementioned reverse osmosis could rapidly reduce a large volume of hydrolysate (about 50-60 L per hydrolysis) to a manageable level for lyophilisation and was also a key step to the subsequent scaleup. Besides, the reverse osmosis could remove the bulk of inorganic salts in the fractions and result in a highly pure final product containing only few amount of inorganic salts.

Lastly, the *Pichia holstii* NRRL Y-2448 culture fermentation hydrolyzed purified phosphomannan components was sulfonated to obtain active constituents of the pharmaceutical composition of the invention. The purified phosphomannan (478 g) obtained from the above was first mixed with Dimethylformamide (DMF, 10 L), sulphur trioxide pyridine complex (3.78 kg) added, the mixture stirred at 25 °C for 3 days, and then a thick oil product were isolated. After removal of dimethylformamide, the residue was washed 3 times with ethanol (1 L) and then dissolved in approximate 3 L of water. The solution was adjusted to pH 9.5 with 5 L of 1 M NaOH, and extracted 3 times with dichloromethane (3 L) to remove released pyridine. The aqueous phase was diluted with water and decolorized with a charcoal filter (Cuno R53S). The solution was diluted with water to 20 L and diafiltered against eight diavolumes of 1 M NaCl solution, and further diafiltered against purified water until the conductivity of the permeate was < 0.2 mS / cm. Finally, the solution was concentrated to 6 L by reverse osmosis, filtered through a membrane with a pore size of 0.2 µm and lyophilised to afford 760 g of a white, hygroscopic, amorphous solid, i.e., the active constituents of the pharmaceutical composition of the invention.

### Validation of the Pharmaceutical Composition of the Invention

The components (constituents) contained in the active constituents of the pharmaceutical composition of the invention were analyzed and validated with Liquid Chromatography / Electrospray Ionization - Fourier transform mass spectrometry, LC/ESI-FTMS. The conditions of detection and analyses are described below.

High performance liquid chromatography (HPLC) set conditions: A HPLC system composed of Shimadzu LC-20ATvp Shimadzu LC-20ADvp pumps, Shimadzu SIL-20AC autosampler and Shimadzu SCL-20A System Controller; UV Detector: Shimadzu SPD-20AV Detector, wave length 280 nm; Data System: Xcalibur 2.0.7; HPLC columns: ACE3, C18-AR, 4.6 x 150 mm; Guard column: ACE3, C18, 3.0µm, 3.2 x 10mm; Column temperature: the surrounding environment's temperature; Autosampler temperature 4°C; Mobile phase A: a mixture of water and methanol (8:2 v/v) containing 5mM dibutyl ammonium acetate; Mobile phase B: a mixture of water and methanol (1:9 v/v) containing 5mM dibutyl ammonium acetate. Gradient elution is shown in Table 1.

**Table 1, Gradient Elution HPLC.**

| Time (min) | Flow rate (mL/min) | Mobile phase A ratio | Mobile phase B ratio |
|---|---|---|---|
| 0 | 0.7 | 70% | 30% |
| 40 | 0.7 | 0 | 100% |
| 45 | 0.7 | 0 | 100% |
| 47 | 0.7 | 70% | 30% |
| 62 | 0.7 | 70% | 30% |

Mass Spectrometer set conditions: Mass Spectrometer: Thermo LTQ Orbitrap XL, Data system: Xcalibur 2.0.7; Ionization mode: electrospray ionization in negative ionization mode; Ion spray voltage 4.5kV; Capillary temperature 350°C, Capillary voltage -18V; tube lens -100V, Sheath gas flow 60 units, Auxiliary gas flow 30 units, Sweep gas flow 5 units; Helium for gas collision.

The results of the analyses of the components contained in the active constituents of the pharmaceutical composition of the invention are shown in Table 2.

**Table 2: Components (Constituents) in active constituents of the composition, and their Structures, Chemical Formula and Molecular Weights.**

| Name | Structure | | Formula (Molecular weight) |
|---|---|---|---|
| Component 1 | 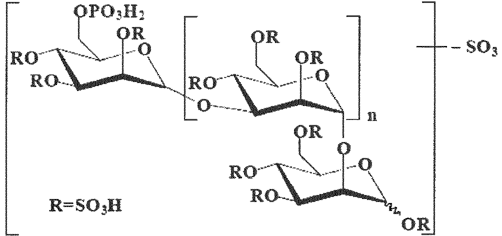 | n=0 | C₁₂H₂₃O₃₂PS₆ (901.8229) |
| Component 3 | | n=1 | C₁₈H₃₃O₄₆PS₉ (1303.7461) |
| Component 5 | | n=2 | C₂₄H₄₃O₆₀PS₁₂ (1705.6694) |
| Component 7 | | n=3 | C₃₀H₅₃O₇₄PS₁₅ (2107.5927) |
| Component 2 | 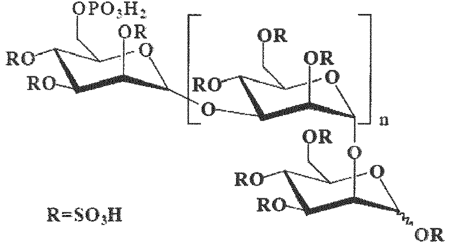 | n=0 | C₁₂H₂₃O₃₅PS₇ (981.7797) |
| Component 4 | | n=1 | C₁₈H₃₃O₄₉PS₁₀ (1383.7030) |
| Component 6 | | n=2 | C₂₄H₄₃O₆₃PS₁₃ (1785.6262) |
| Component 8 | | n=3 | C₃₀H₅₃O₇₇PS₁₆ (2187.5495) |

As shown in Table 2, the active constituents of the pharmaceutical composition of the invention comprise a total of 8 components: components 1 to 8, excluding their isomers. The basic structure of the components is an oligosaccharide composed of 2∼ 5 mannose units linked via 1→3 and/or 1→2 glycosidic bond. Furthermore, the mannose units are linked via 1→3 glycosidic bond except the end unit, which is linked via 1→2 glycosidic bond. Referring to chemical structures in Table 1, when n = 0, the compound represents a disaccharide; when n = 1, the compound represents a trisaccharide; when n = 2, the compound represents a tetrasaccharide, and when n = 3 the compound represents a pentasaccharide.

Based on the basic structures above, the hydrogen (H) in the -OH group at C6 of the first mannose unit in each component of the active constituents of the pharmaceutical composition of the invention was replaced by PO₃H₂ or a salt thereof. The hydrogen (H) in the 3n+6 or 3n+7 -OH groups out of the remaining 3n+7 -OH groups were replaced by SO₃H or a salt thereof. Referring to the structures in Table 2, Components 1, 3, 5 and 7 were disaccharides, trisaccharides, tetrasaccharides and pentasaccharides with hydrogen (H) in 3n+6 -OH groups being replaced by SO₃H or a salt thereof, respectively. That is, except the -OH group at C6 of the first mannose unit, only one -OH group was not replaced by SO₃H or a salt thereof. Components 2, 4, 6 and 8 were disaccharides, trisaccharides, tetrasaccharides and pentasaccharides with hydrogen (H) in 3n+7 -OH groups being replaced by SO₃H or a salt thereof, respectively. That is, except the -OH group at C6 of the first mannose unit, all of the -OH group were replaced by SO₃H or a salt thereof.

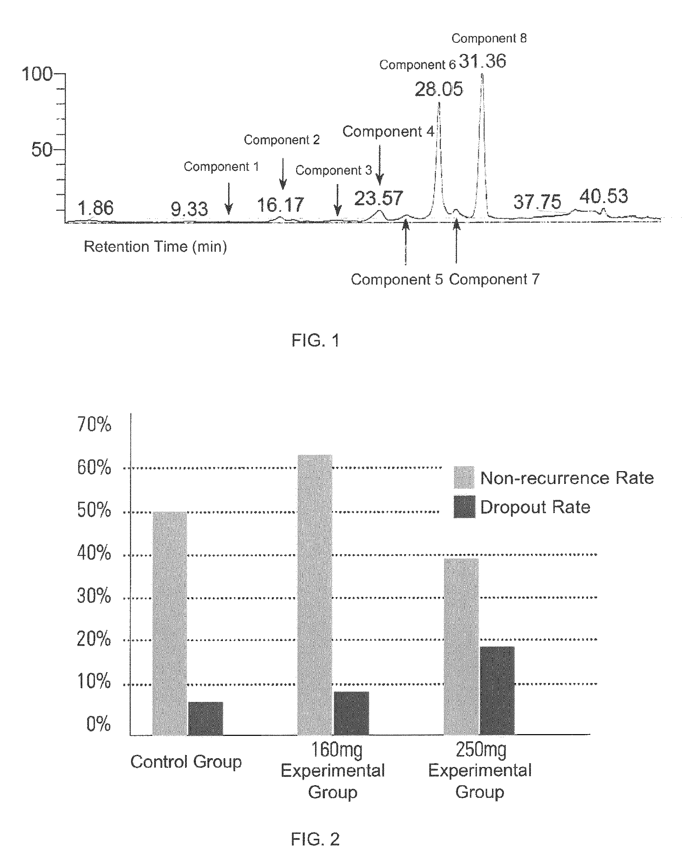

FIG. 1 shows the results of LC/ESI-FTMS total ion chromatogram (TIC) analysis of the active constituents of the pharmaceutical compositions of the invention. As indicated in FIG. 1, among the active constituents of the pharmaceutical composition of the invention, components 8 and 6 were primary constituents. That is, the compound of formula (I) having n =2 or 3 and 3n+7 of R representing SO₃H was the primary constituent. In addition, FIG. 1 also indicates that among active constituents of the pharmaceutical composition of the invention, component (or constituent) 8 had the highest relative content. That is, the compound of formula (I) having n=3 and 3n+7 of R representing SO₃H had the highest relative content.

FIG. 1 also indicates that in addition to the components from 1 to 8, the active constituents of the pharmaceutical composition of the invention might also contain other constituents, e.g., those components corresponding to the retention times of 1.86 min, 9.33 min, 37.75 min and 40.53 min.

The data for each of the components based on the LC/ESI-FTMS total ion chromatogram (TIC) analyses, which included apex retention time, peak area, peak area percentage, peak height, and peak height percentage, are shown in Table 3 below.

**Table 3: Relevant Data on Each Component of the Pharmaceutical Composition of the Invention Based on LC/ESI-FTMS analyses**

| Name | Apex Retention Time (min) | Peak Area | Peak Area Percentage | Peak Height | Peak Height Percentage |
|---|---|---|---|---|---|
| Component 1 | 12.31 | 699406 | 0.09% | 25711 | 0.1% |
| Component 2 | 16.17 | 21279900 | 2.89% | 422185 | 1.72% |
| Component 3 | 20.55 | 10764686 | 1.46% | 159934 | 0.65% |
| Component 4 | 23.57 | 50971043 | 6.92% | 942431 | 3.84% |
| Component 5 | 25.62 | 30026265 | 4.08% | 477234 | 1.95% |
| Component 6 | 28.05 | 249954793 | 33.94% | 9663862 | 39.39% |
| Component 7 | 29.38 | 17374916 | 2.36% | 532147 | 2.17% |
| Component 8 | 31.36 | 355469535 | 48.26% | 12308532 | 50.17% |

The data on the peak area percentage of each component in Table 3 indicate that components 6 and 8 were the primary constituents in the active constituents of the pharmaceutical composition of the invention. The peak areas of components 6 and 8 were 82.2% of the total peak areas. That is, in the compound of formula (I) the compound having n=2 or 3 and 3n+7 of R representing SO₃H was the primary constituent. In addition, Table 3 also indicates in the active constituents of the pharmaceutical composition of the invention, component 8 had the highest relative content. Its peak area was 48.26% of the total peak areas. That is, the compound of formula (I) having n=3 and 3n+7 of R representing SO₃H had the highest relative content. Based on the replacement of -OH groups by SO₃H, the results in Table 3 indicate that in the compound of formula (I), compounds having all of the -OH group being replaced by SO₃H or a salt thereof except the -OH group at C6 of the first mannose unit, namely components 2, 4, 6 and 8, had a sum of peak area of 92.01% of the total peak areas. On the other hand, in the compound of formula (I), compounds having 3n+6 -OH groups out of 3n+7 -OH groups being replaced by SO₃H or a salt thereof, namely components 1, 3, 5 and 7, had a sum of peak area of 7.99% of the total peak areas. Therefore, compounds having all 3n+7 -OH groups being replaced by SO₃H were the primary components in the active constituents of the pharmaceutical composition of the invention.

### The Utility of the Pharmaceutical Composition of the Invention

The pharmaceutical composition of the invention is currently used as an adjunct therapy in liver cancer treatment. In a preferred embodiment, the pharmaceutical composition of the invention is for use in inhibiting recurrence, aggravation and metastasis of liver cancer. Furthermore, the clinical effect of the pharmaceutical composition of the invention is to delay time to recurrence, or to decrease recurrence rate of liver cancer patients after liver resection. The following describes working examples concerning the indications, pharmacological effects, effective dosage, directions, pharmacological experimental methods and results of the pharmaceutical composition of the invention and proofs of the utility of the invention.

The suitable indication for the pharmaceutical composition of the invention is liver cancer. The composition may be combined with at least one of other therapies such as embolization, targeted therapy, chemotherapy, radiotherapy or hepatectomy, for use in inhibiting the recurrence, aggravation and metastasis of liver cancer. In a preferred embodiment, the pharmaceutical composition of the invention is suitable for use in a liver cancer patient after liver resection. The pharmaceutical composition of the invention exerts its pharmacological effects in inhibiting liver cancer metastasis and liver cancer angiogenesis by inhibition of heparanase and vascular growth factor activities, and achieves therapeutic effects in inhibition of recurrence, aggravation and metastasis of liver cancer. In a preferred embodiment, the safety, preliminary effectiveness and effective dosage of the pharmaceutical composition of the invention have been validated by human clinical trials, and the composition was effective in delaying recurrence time or decrease recurrence rate in liver cancer patients. The clinical trials were multi-center, randomized and parallel-group trials.

The route of administration in the clinical use of the pharmaceutical composition of the invention is via injection. Water may be a solvent due to a high water solubility. Suitable solvents for injections include but not limited to sterile water, sterile physiological saline solution with water as a solvent or a glucose solution, etc., without exclusion of other oil solvents. In a preferred embodiment, isotonic normal physiological saline is used as a solvent.

The pharmaceutical composition of the invention is formulated in a dosage unit form for easy administration and dosage uniformity. Each dosage unit contains a specified amount of the active constituents mixed with a pharmaceutical acceptable diluent, excipient or vehicle, and provides anticipated therapeutic effects.

The active constituents of the pharmaceutical composition of the invention have a water solubility of greater than 400 mg/ml. Thus, 160 mg of the active constituents of the pharmaceutical composition of the invention may be completely dissolved in 0.4 ml of water. In a preferred embodiment, every dosage unit contains 215 mg of the active constituents and 1 ml of a physiological saline solution, the concentration of which after mixing is 200 mg/ml. Injecting 0.8 ml of the above is equivalent to 160 mg of the active constituents of the pharmaceutical composition of the invention.

The efficacy/safety population used in clinical trials was Intent-to-treat (ITT) population. In Intent-to-treat population, all the qualified, randomly assigned patients were included in analysis, but excluding those who had not taken the experimental drug or had no record after the random assignment.

In the aforementioned clinical trial, 172 liver cancer patients who had receive liver resection were randomly divided into three groups, one group (58 patients) as a untreated control group, one group (57 patients) as an experimental group treated with 160 mg daily, the other group (57 patients) as an experimental group treated with 250 mg daily of the active constituent of the pharmaceutical composition of the invention. Patients in the experimental group started treatment 4 to 6 weeks after liver resection. Each treatment cycle was 4 weeks. Patients were dosed s.c. for 4 consecutive days per week in the first 3 weeks of each cycle, but not dosed in the fourth week of each cycle. After 9 continuous treatment cycles (total 36 weeks), patients were followed up for 12 weeks (follow-up period). Patients in the two experimental groups were routinely examined and monitored every 4 weeks during the 36-week-treatment period and checked every 6 weeks during the 12-week-follow-up period. Patients in the untreated group were not given any control drug or placebo, and were routinely examined and monitored every 6 weeks during the 48 weeks of the test period.

Before the start of each cycle, participants were subject to medical history confirmation, physical examinations, concomitant medication status confirmation, and routine laboratory tests including α-fetoprotein (AFP) test. All participants received abdominal ultrasound examinations monthly. Abdominal CT scans were conducted on the first day of the 4th and the 7th cycles. In addition, participants who were suspected of having liver cancer recurrence were subject to abdominal CT scans. Chest X-ray examinations, bone scans and head CT scans were also performed on the first day of the 4th and the 7th cycles to monitor extra-hepatic tumor recurrence.

Serum α-fetoprotein tests and abdominal ultrasound examinations were routinely used as a basis of a preliminary evaluation for liver cancer recurrence. When the serum α-fetoprotein concentration increased or an abdominal CT scan indicated a possibility of new tumor formation, it was necessary to conduct an abdominal CT scan to examine the vasculature of the recurrent tumor. When the tumor showed typical hypervascularity in the arterial phase of the CT scan and the contrast medium was fast washed out in the venous phase, the focus of infection was defined as liver cancer(HCC) recurrence. If the focus of infection in the participant had received a biopsy or surgical resection, liver cancer recurrence might also be confirmed by histological examinations.

The primary efficacy endpoint in the clinical trials above was non-recurrence rate of HCC at the completion of the trial. The secondary efficacy endpoint was the time to first recurrence calculated from the date of randomization to the date of confirmed tumor recurrence by CT scan or histological examinations.

The data of the clinical trial are the following: 58 out of 58 patients in the control group were able to be included in the Intent-to-treat analyses, 26 of them had liver cancer recurrence at the completion of the trial, which was about 45%; 29 of them did not have liver cancer recurrence at the completion of the trial, which was about 50%; 3 of them had dropped out in the middle of the trial, which was about 5%. In the experimental group treated with 160mg/day of the active constituent of the pharmaceutical composition of the invention, 56 out of 57 patients were able to be included in the Intent-to-treat analyses, 16 of them had liver cancer recurrence at the completion of the trial, which was about 29%; 35 of them had no recurrence at the completion of the trial, which was about 63%; 5 of them dropped out in the middle of the trial, which was about 9%. In the experimental group treated with 250mg/day of the active constituent of the pharmaceutical composition of the invention, 54 out of 57 patients were able to be included in the Intent-to-treat analyses, 21 of them had liver cancer recurrence at the completion of the trial, which was about 39%; 22 of them had no recurrence at the completion of the trial, which was about 41%; 11 of them dropped out in the middle of the trial, which was about 20%.

FIG. 2 shows the non-recurrence rate and the dropout rate of the liver cancer patients tested in the control group, the experimental group treated with 160mg/day, and the experimental group treated with 250 mg/day of the active constituent of the pharmaceutical composition of the invention. Referring to FIG. 2, the experimental group treated with 160mg/day of the active constituent of the pharmaceutical composition of the invention showed a non-recurrence rate of 63%, whereas the untreated control group showed a non-recurrence rate of 50%. The statistics indicated there was a difference (P = 0.07). That is, the active constituents of the pharmaceutical composition of the invention were able to decrease liver cancer recurrence in the liver cancer patients after liver resection when periodically treated with a dose of 160mg/day, s.c..

The results of the trial above also indicate that the experimental group treated with 250mg/day of the active constituent of the pharmaceutical composition of the invention did not significantly decrease liver cancer recurrence when compared with the untreated control group.

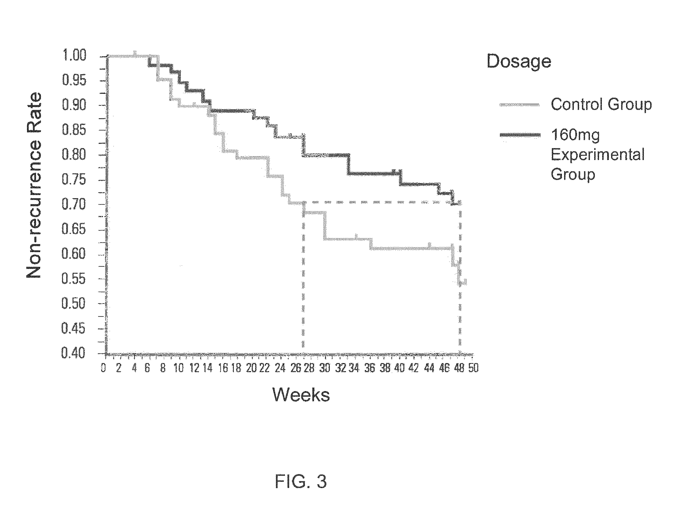

FIG. 3 shows comparisons between the control group and the experimental group treated with 160mg/day of the active constituent of the pharmaceutical composition of the invention for changes in non-recurrence rates in liver cancer patients during the trial period (0∼ 48 weeks). The dashed line represents the time required from the beginning of the trial to the time when 70% of patients did not have recurrence in both groups. Referring to FIG. 3, in the control group the time required from the beginning of the trial to the time of reaching 70% of the participants having non-recurrence was 27 weeks. In the experimental group treated with 160 mg/day of the active constituent of the pharmaceutical composition of the invention, the time required from the beginning of the trial to the time reaching 70% of the participants having non-recurrence was 48 weeks. The results indicated that the active constituents of the pharmaceutical composition of the invention could delay liver cancer recurrence in liver cancer patients after liver resection when periodically treated with a dosage of 160mg/day, s.c..

In addition, other clinical trials indicated that a daily dose of 80mg of the active constituent of the pharmaceutical composition of the invention was also effective in inhibiting the increase in the tumor size, whereas a daily dose of 315mg of the active constituent of the pharmaceutical composition of the invention may cause severe thrombocytopenia.

The foregoing descriptions are mere preferred embodiments of the invention which are not meant to limit the scope of the invention. Any variations and modifications without departing from the spirit and scope of the invention made by any skilled in the art are all encompassed in the appended claims.

## Claims

1. A pharmaceutical composition for use in inhibiting recurrence, aggravation and/or metastasis of liver cancer, comprising a therapeutically effective amount of at least one compound of formula (I) and a pharmaceutically acceptable diluent, excipient or vehicle; 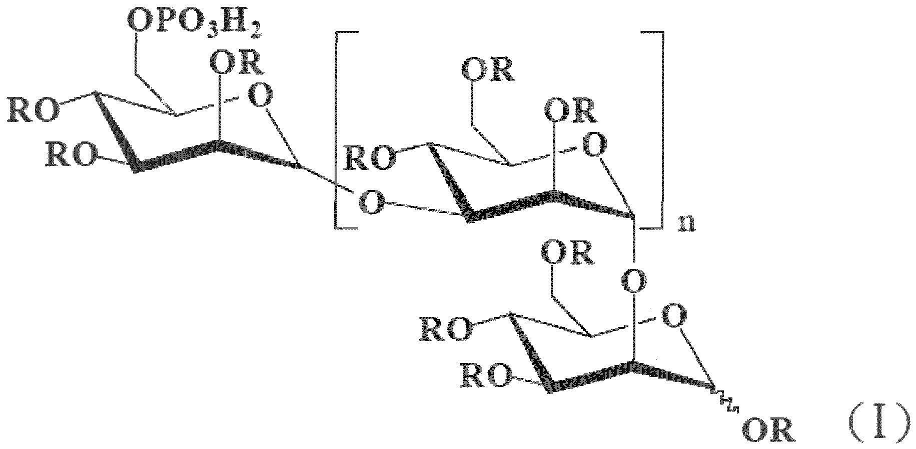 wherein in the compound of formula (I), n is an integer of from 0 to 3, and 3n+6 or 3n+7 of the R groups are SO₃H, and the rest of the R groups are H.

2. The pharmaceutical composition of claim 1, wherein the compound having 3n+7 of the R groups as SO₃H is the primary constituent.

3. The pharmaceutical composition of claim 1, wherein the compound having n equal to 2 or 3 and 3n+7 of the R groups as SO₃H is the primary constituent.

4. The pharmaceutical composition of claim 1, wherein the compound having n equal to 3 and 3n+7 of the R groups as SO₃H has the highest relative content.

5. A pharmaceutical composition as claimed in any one of claims 1 to 4, wherein the therapeutically effective amount is between 80mg to 315mg per day.

6. The pharmaceutical composition of claim 5, wherein the therapeutically effective amount is 160mg per day.

7. The pharmaceutical composition of claim 6, which is in combination with at least one therapy for use in inhibiting recurrence, aggravation or metastasis of liver cancer, wherein the at least one therapy comprises embolization therapy, target drug therapy, chemotherapy, radiation therapy and liver surgical resection.

8. The pharmaceutical composition of claim 7, which is for use in decreasing recurrence rate after the liver surgical resection or prolonging time to recurrence after the liver surgical resection in a liver cancer patient.

9. A method of using the compound of formula (I) as claimed in claim 1 for preparing a medicament for use in inhibiting recurrence, aggravation and/or metastasis of liver cancer, comprising combining a therapeutically effective amount of the compound of formula (I) and a pharmaceutically acceptable diluent, excipient or vehicle, wherein n is an integer of from 0 to 3, and 3n+6 or 3n+7 of the R groups are SO₃H, and the rest of the R groups are H.

10. The method of claim 9, wherein the compound having 3n+7 of the R groups as SO₃H is the primary constituent.

11. The method of claim 9, wherein the compound having n equal to 2 or 3 and 3n+7 of the R groups as SO₃H is the primary constituent.

12. The method of claim 9, wherein the compound having n equal to 3 and 3n+7 of the R groups as SO₃H has the highest relative content.

13. A method as claimed in any one of claims 9 to 12, wherein the pharmaceutically acceptable diluent, excipient or vehicle is water or a solution containing water as a solvent.

14. The method of claim 13, wherein the pharmaceutically acceptable diluent, excipient or vehicle is a physiological saline solution or a glucose solution.
